# EUROPEAN PATENT APPLICATION

(11) **EP 4 364 665 A1**
(43) Date of publication of application: **08.05.2024**
(21) Application number: 22832958.7
(22) Date of filing: 22.06.2022
(51) Int. Cl.: A61B 6/00, A61B 6/03, G06T 7/00

(54) **DISEASE LABEL CREATION DEVICE, METHOD, AND PROGRAM, LEARNING DEVICE, AND DISEASE DETECTION MODEL**

(30) Priority: 29.06.2021 JP 2021107709
(71) Applicant: FUJIFILM Corporation, Tokyo 106-8620 (JP)
(72) Inventor: HIASA, Yuta, Tokyo 106-8620 (JP)
(74) Representative: Hughes, Andrea Michelle
(86) International application number: PCT/JP2022/024843
(87) International publication number: WO 2023/276810

(57) **Abstract**

Provided are a disease label creation device, a disease label creation method, a disease label creation program, a learning device, and a disease detection model that can create a disease label for a simple X-ray image at a low annotation cost. An information acquisition unit (28) of a first processor of a disease label creation device (10-1) acquires a simple X-ray image (1), a three-dimensional CT image (2) paired with the simple X-ray image (1), and a three-dimensional first disease label extracted from the CT image (2). A registration processing unit (30) of the first processor performs registration between the simple X-ray image (1) and the CT image (2). A disease label converter (40) of the first processor converts the first disease label into a two-dimensional second disease label corresponding to the simple X-ray image on the basis of a result of the registration by the registration processing unit (30) to create a disease label (3-1).

## Description

### BACKGROUND OF THE INVENTION

### 1. Field of the Invention

The present invention relates to a disease label creation device, a disease label creation method, a disease label creation program, a learning device, and a disease detection model, and particularly relates to a technique that creates a disease label used to train a disease detection model.

### 2. Description of the Related Art

In simple X-ray imaging, a subject is irradiated with X-rays from an X-ray source, and an image (hereinafter, referred to as a "simple X-ray image") corresponding to an X-ray dose transmitted through the subject is captured. A specialized doctor visually recognizes, for example, the presence or absence and size of a disease from the simple X-ray image. However, since the disease is superimposed on organs or bones in the simple X-ray image, it is not easy to visually recognize the disease.

Therefore, it is desired to construct a disease detection model for detecting a disease from the simple X-ray image in order to support interpretation of the simple X-ray image by the specialized doctor.

In a case in which this type of disease detection model is constructed, it is common to prepare a large amount of training data consisting of a one-to-one pair of a simple X-ray image and a disease label and to optimize (learn) parameters of the disease detection model using the training data.

Nam, Ju Gang, et al. "Development and validation of deep learning-based automatic detection algorithm for malignant pulmonary nodules on chest radiographs.", Radiology, 2019. discloses a technique in which, in a case in which region information (disease label) of a lung nodule for a simple X-ray image of a patient is created, a doctor manually creates the disease label with reference to a computed tomography (CT) image of the same patient.

In addition, Zhang, Yue, et al. "Task driven generative modeling for unsupervised domain adaptation: Application to x-ray image segmentation.", MICCAI, 2018. discloses a technique that utilizes a disease label (label information) of a CT image for a simple X-ray image. In the technique disclosed in Zhang, Yue, et al. "Task driven generative modeling for unsupervised domain adaptation: Application to x-ray image segmentation.", MICCAI, 2018., a CT image is projected and converted into a digital reconstructed radiograph (DRR) image, and a disease detection model trained with the DRR image and projected label information is applied to the simple X-ray image.

### SUMMARY OF THE INVENTION

As described in Nam, Ju Gang, et al. "Development and validation of deep learning-based automatic detection algorithm for malignant pulmonary nodules on chest radiographs.", Radiology, 2019., in a case in which a disease label for a simple X-ray image is manually created while referring to a computed tomography (CT) image of the same patient, there is a problem in that a lot of effort and time is required and an annotation cost increases. In addition, in a case in which an annotation at a level that can be visually recognized in the CT image, but is not capable of being visually recognized in the simple X-ray image is reflected in training, the annotation may be noise in the training of the disease detection model.

Further, as described in Zhang, Yue, et al. "Task driven generative modeling for unsupervised domain adaptation: Application to x-ray image segmentation.", MICCAI, 2018., the DRR image created from the CT image has a lower resolution than the simple X-ray image. Therefore, the disease detection model trained with the DRR image has a problem that it can recognize only a global region, such as an anatomical region, but is not suitable for detecting a small disease such as a lung nodule.

The present invention has been made in view of these circumstances, and an object of the present invention is to provide a disease label creation device, a disease label creation method, a disease label creation program, a learning device, and a disease detection model that can create a disease label for a simple X-ray image at a low annotation cost and use the created disease label as training data for a disease detection model to construct a disease detection model having a high reliability in detection of diseases.

In order to achieve the above object, according to a first aspect of the present invention, there is provided a disease label creation device comprising a first processor. The first processor is configured to execute: an information acquisition process of acquiring a simple X-ray image, a three-dimensional CT image paired with the simple X-ray image, and a three-dimensional first disease label extracted from the CT image; a registration process of performing registration between the simple X-ray image and the CT image; and a conversion process of converting the first disease label into a two-dimensional second disease label corresponding to the simple X-ray image on the basis of a result of the registration.

In the first aspect of the present invention, the registration between the simple X-ray image and the CT image is performed, and the three-dimensional first disease label extracted from the CT image is converted into the two-dimensional second disease label corresponding to the simple X-ray image on the basis of the result of the registration. This makes it possible to automatically create a disease label for the simple X-ray image and to reduce an annotation cost. Further, modalities and the postures of a patient during imaging are different in the simple X-ray image and the CT image. However, since the three-dimensional first disease label is converted into the two-dimensional second disease label corresponding to the simple X-ray image according to the registration between the two images, it is possible to generate a disease label (second disease label) in which a pixel-level annotation has been reflected.

According to a second aspect of the present invention, in the disease label creation device, preferably, the registration process includes: a process of projecting the CT image to create a pseudo X-ray image; and a process of performing registration between the simple X-ray image and the pseudo X-ray image.

According to a third aspect of the present invention, in the disease label creation device, preferably, the registration process includes: a process of extracting a two-dimensional anatomical landmark from the simple X-ray image; a process of extracting a three-dimensional anatomical landmark corresponding to the anatomical landmark from the CT image; a process of projecting the three-dimensional anatomical landmark; and a process of performing registration between the two-dimensional anatomical landmark and an anatomical landmark after the projection process. For example, a rib with a high resolution in the simple X-ray image is considered as the anatomical landmark.

According to a fourth aspect of the invention, in the disease label creation device, preferably, the registration process includes: a process of extracting a two-dimensional anatomical region of interest from the simple X-ray image; a process of extracting a three-dimensional anatomical region of interest corresponding to the anatomical region of interest from the CT image; a process of projecting the three-dimensional anatomical region of interest; and a process of performing registration between a contour of the two-dimensional anatomical region of interest and a contour of an anatomical region of interest after the projection process. Examples of the anatomical region of interest include regions of a lung field, a thorax, a heart, and an aorta.

According to a fifth aspect of the present invention, in the disease label creation device, preferably, the registration process includes: a process of three-dimensionally restoring the simple X-ray image; and a process of performing registration between the CT image and the three-dimensionally restored simple X-ray image.

According to a sixth aspect of the present invention, in the disease label creation device, preferably, the first processor is configured to execute a first reliability calculation process of calculating a first reliability for the second disease label.

According to a seventh aspect of the present invention, in the disease label creation device, preferably, in the first reliability calculation process, a visibility of a second disease region corresponding to the second disease label with respect to a normal region of the simple X-ray image is calculated using at least one of statistics of pixel values of a normal region and a first disease region of the CT image corresponding to the first disease label or a shape feature of the first disease region of the CT image, and the first reliability is calculated from the calculated visibility. In a case in which the difference between the statistics (for example, the average values) of the pixel values of the first disease region and the normal region of the CT image is large, the visibility of the second disease region is high. In addition, for example, in a case in which the first disease region has a shape that is long in a projection direction from the shape feature of the first disease region of the CT image, the difference between X-ray absorption amounts of the first disease region and the normal region is large, and the visibility of the second disease region is high.

According to an eighth aspect of the present invention, in the disease label creation device, preferably, in the information acquisition process, information of an anatomical region in the CT image is acquired. Preferably, in the first reliability calculation process, a visibility of a second disease region corresponding to the second disease label with respect to a normal region of the simple X-ray image is calculated on the basis of superimposition of the anatomical region and a first disease region of the CT image corresponding to the first disease label in a projection direction, and the first reliability is calculated from the calculated visibility. In a case in which the first disease region of the CT image is superimposed on the anatomical region (for example, a bone region) in the projection direction, an X-ray transmission amount of a disease region (second disease region) of the simple X-ray image corresponding to the first disease region is reduced due to the bone region, and the visibility of the disease region is low.

According to a ninth aspect of the present invention, in the disease label creation device, preferably, the first disease label is a label automatically detected from the CT image. Preferably, in the information acquisition process, an interpretation report corresponding to the simple X-ray image or the CT image is acquired. Preferably, in the first reliability calculation process, the first reliability is calculated on the basis of a rate of match between the first disease label and content described in the interpretation report. This makes it possible to reflect the results of image diagnosis by a specialized doctor in the first reliability.

According to a tenth aspect of the present invention, in the disease label creation device, preferably, the first processor is configured to calculate a degree of success of the result of the registration. Preferably, in the first reliability calculation process, the first reliability is calculated on the basis of the degree of success.

According to an eleventh aspect of the present invention, in the disease label creation device, preferably, the first disease label is a label automatically detected from the CT image. Preferably, in the first reliability calculation process, a low first reliability is given to the second disease label of a region having different imaging ranges in the simple X-ray image and the CT image forming the pair.

According to a twelfth aspect of the present invention, in the disease label creation device, preferably, in the registration process, the registration is performed by adjusting a solution space in the registration between the simple X-ray image and the CT image forming the pair associated with a patient, depending on the patient.

According to a thirteenth aspect of the present invention, preferably, the disease label creation device further comprises a database of a statistical deformation model for each patient feature information item. Preferably, the registration process includes: a process of selecting a corresponding statistical deformation model from the database on the basis of patient feature information of the patient corresponding to the simple X-ray image and the CT image forming the pair; and a process of performing non-rigid registration between the simple X-ray image and the CT image using the selected statistical deformation model.

For example, for a patient with a disorder in which the ribs do not move, in a case in which the positions of the ribs in the simple X-ray image and the CT image of the patient are registered, a statistical deformation model corresponding to the patient (disorder) is selected, and the non-rigid registration between the simple X-ray image and the CT image is performed using the selected statistical deformation model.

According to a fourteenth aspect of the present invention, in the disease label creation device, preferably, in the information acquisition process, an image-level third disease label of the CT image is acquired, and the first processor is configured to give the second disease label and the third disease label to the simple X-ray image. For example, a label indicating a class classification (a lung nodule, a lung tumor, or the like) of a disease can be given as the image-level third disease label of the CT image.

According to a fifteenth aspect of the present invention, in the disease label creation device, preferably, in the information acquisition process, an image-level third disease label of the CT image is acquired, and the first processor is configured to: determine whether the result of the registration is a success or a failure; select the second disease label in a case in which it is determined that the result is a success and select the third disease label in a case in which it is determined that the result is a failure; and give the selected second disease label or the selected third disease label to the simple X-ray image.

According to a sixteenth aspect of the present invention, there is provided a disease label creation method executed by a processor. The disease label creation method comprises: a step of acquiring a simple X-ray image, a three-dimensional CT image paired with the simple X-ray image, and a three-dimensional first disease label extracted from the CT image; a step of performing registration between the simple X-ray image and the CT image; and a step of converting the first disease label into a two-dimensional second disease label corresponding to the simple X-ray image on the basis of a result of the registration.

According to a seventeenth aspect of the present invention, there is provided a disease label creation program causing a computer to implement: a function of acquiring a simple X-ray image, a three-dimensional CT image paired with the simple X-ray image, and a three-dimensional first disease label extracted from the CT image; a function of performing registration between the simple X-ray image and the CT image; and a function of converting the first disease label into a two-dimensional second disease label corresponding to the simple X-ray image on the basis of a result of the registration.

According to an eighteenth aspect of the present invention, there is provided a learning device comprising a second processor. The second processor is configured to: execute a learning process of training a disease detection model, using first training data consisting of the simple X-ray image and the second disease label created by the disease label creation device according to the first aspect of the second aspect and converging a first error between an output of the disease detection model and the second disease label.

According to the eighteenth aspect of the present invention, the disease detection model is trained using the first training data (the first training data obtained at a low annotation cost) consisting of the simple X-ray image and the second disease label created by the disease label creation device according to the first aspect or the second aspect. Therefore, it is possible to train the disease detection model at a low cost. Further, since the second disease label is created on the basis of the CT image that is paired with the simple X-ray image (same patient) and is easier to visually recognize, the first training data consisting of the second disease label and the simple X-ray image is good training data with little noise, and it is possible to construct a disease detection model with high detection accuracy.

According to a nineteenth aspect of the present invention, there is provided a learning device comprising a second processor. The second processor is configured to: in a case in which a learning process of training a disease detection model, using second training data consisting of the simple X-ray image, the second disease label created by the disease label creation device according to any one of the sixth to sixteenth aspects, and the first reliability and converging a first error between an output of the disease detection model and the second disease label is performed, execute the learning process of adjusting the first error according to the first reliability to train the disease detection model.

According to the nineteenth aspect of the present invention, not only the second disease label but also the first reliability based on the visibility of the disease is used, and the first error is adjusted according to the first reliability to train the disease detection model. Therefore, it is possible to reduce the influence of annotation noise at a level that can be visually recognized in the CT image, but is not capable of being visually recognized or is difficult to visually recognize in the simple X-ray image.

According to a twentieth aspect of the present invention, in the learning device, preferably, in the information acquisition process, an image-level third disease label of the CT image is acquired. Preferably, the first processor is configured to give the second disease label and the third disease label to the simple X-ray image, and the second processor is configured to execute a learning process of converging a second error between the output of the disease detection model and the third disease label, using the simple X-ray image to which the third disease label has been given as third training data.

According to a twenty-first aspect of the present invention, in the learning device, preferably, the second processor is configured to execute a learning process of directing the disease detection model to output a disease detection result indicating a disease region included in the simple X-ray image and a second reliability of the disease detection result. This makes it possible for the disease detection model to detect a reliability (second reliability) for the second disease label.

According to a twenty-second aspect of the present invention, in the learning device, preferably, the second processor is configured to adjust the first error of the disease region, of which the second reliability output from the disease detection model is low and which is false positive, to a large value and adjust the first error of the disease region, of which the second reliability is low and which is false negative, to a small value.

According to a twenty-third aspect of the present invention, in the learning device, preferably, the second processor is configured to, in a case in which a learning process of integrating the first reliability calculated by the first reliability calculation process and the second reliability output from the disease detection model to generate a third reliability and converging a first error between an output of the disease detection model and the second disease label, execute the learning process of adjusting the first error according to the third reliability to train the disease detection model.

According to a twenty-fourth aspect of the present invention, there is provided a disease detection model trained by the learning device according to any one of the eighteenth to twenty-fourth aspects. The disease detection model receives any simple X-ray image as an input image, detects a disease label from the input simple X-ray image, and outputs the disease label.

According to the present invention, it is possible to create a disease label for a simple X-ray image at a low annotation cost, to use the created disease label as training data for a disease detection model, and to construct a disease detection model with a high reliability in detection of disease.

### BRIEF DESCRIPTION OF THE DRAWINGS

Fig. 1 is a block diagram illustrating an embodiment of a hardware configuration of a disease label creation device and a learning device according to the present invention.
Fig. 2 is a functional block diagram illustrating a first embodiment of the disease label creation device according to the present invention.
Fig. 3 is a block diagram illustrating a first embodiment of a registration processing unit illustrated in Fig. 2.
Fig. 4 is a functional block diagram illustrating a second embodiment of the disease label creation device according to the present invention.
Fig. 5 is a functional block diagram illustrating a first embodiment of the learning device according to the present invention.
Fig. 6 is a functional block diagram illustrating a second embodiment of the learning device according to the present invention.
Fig. 7 is a diagram illustrating a first embodiment of a disease detection model according to the present invention.
Fig. 8 is a diagram illustrating a second embodiment of the disease detection model according to the present invention.
Fig. 9 is a flowchart illustrating an embodiment of a disease label creation method according to the present invention.

### DESCRIPTION OF THE PREFERRED EMBODIMENTS

Hereinafter, preferred embodiments of a disease label creation device, a disease label creation method, a disease label creation program, a learning device, and a disease detection model according to the present invention will be described with reference to the accompanying drawings.

### [Hardware Configuration of Disease Label Creation Device and Learning Device]

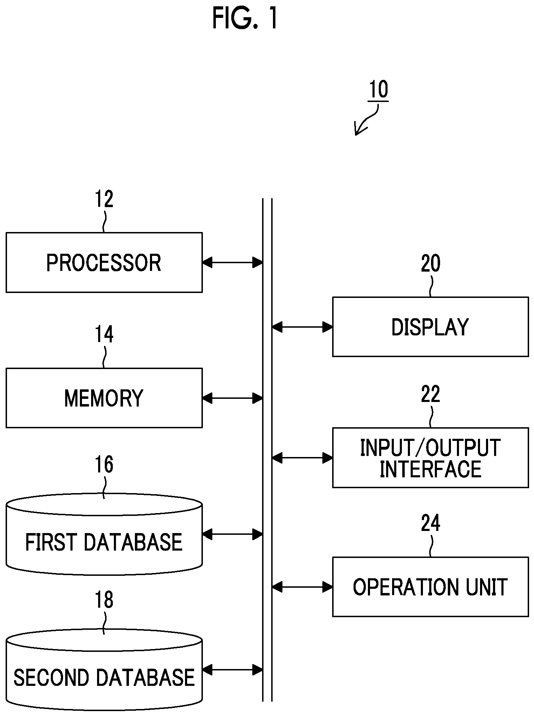

Fig. 1 is a block diagram illustrating an embodiment of a hardware configuration of the disease label creation device and the learning device according to the present invention. The disease label creation device according to this example includes functions of the learning device. However, the disease label creation device and the learning device may be physically separate devices.

A disease label creation device 10 illustrated in Fig. 1 can be configured by a personal computer, a workstation, or the like and comprises a processor 12, a memory 14, a first database 16, a second database 18, a display 20, an input/output interface 22, an operation unit 24, and the like.

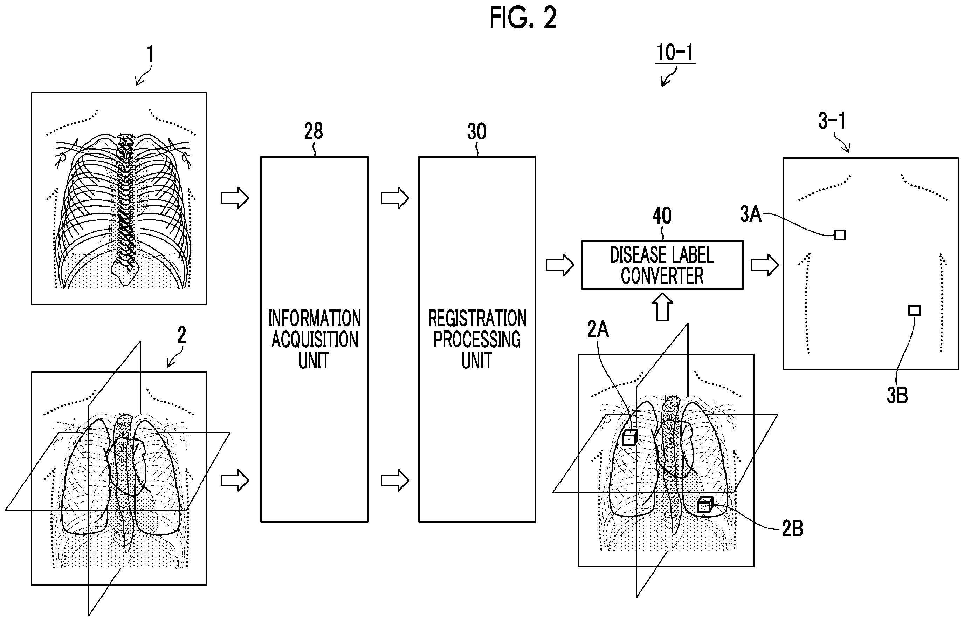

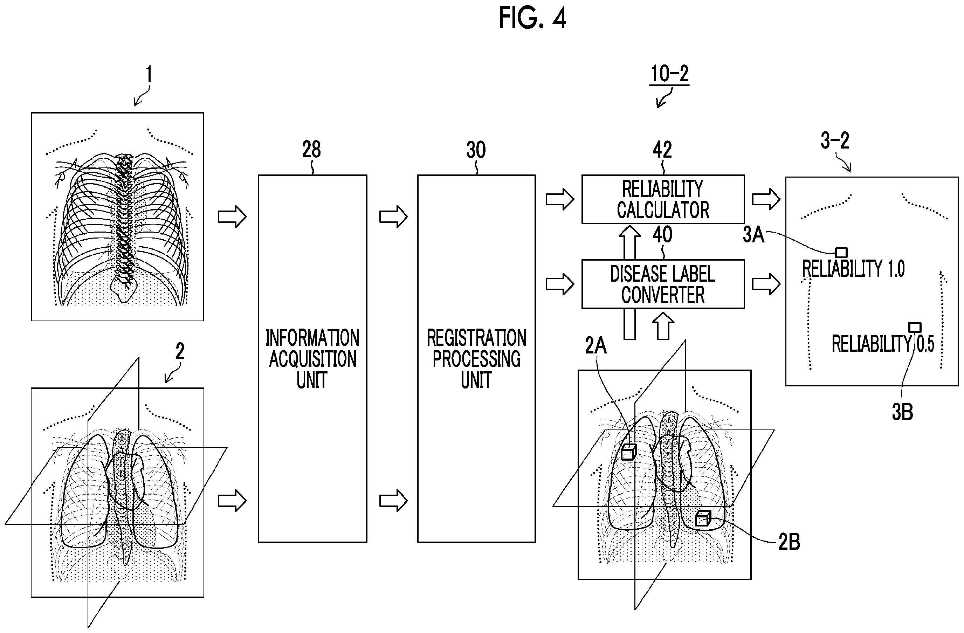

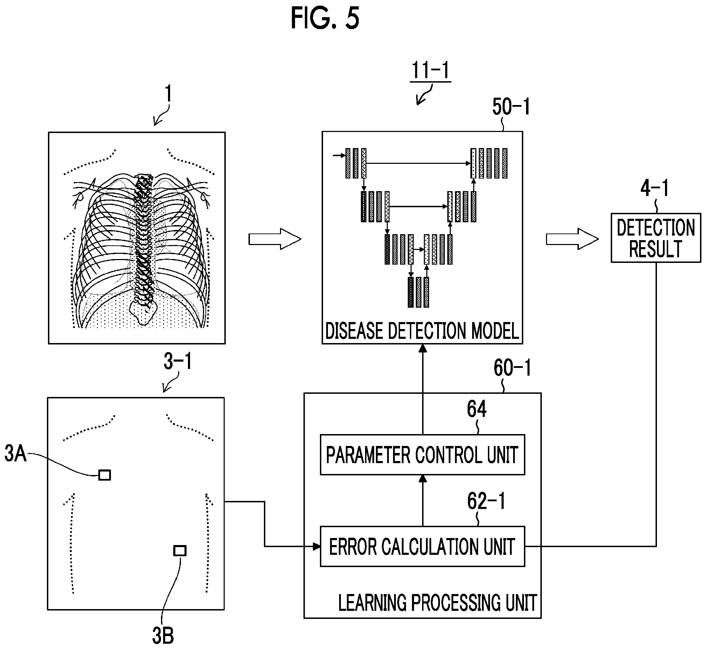

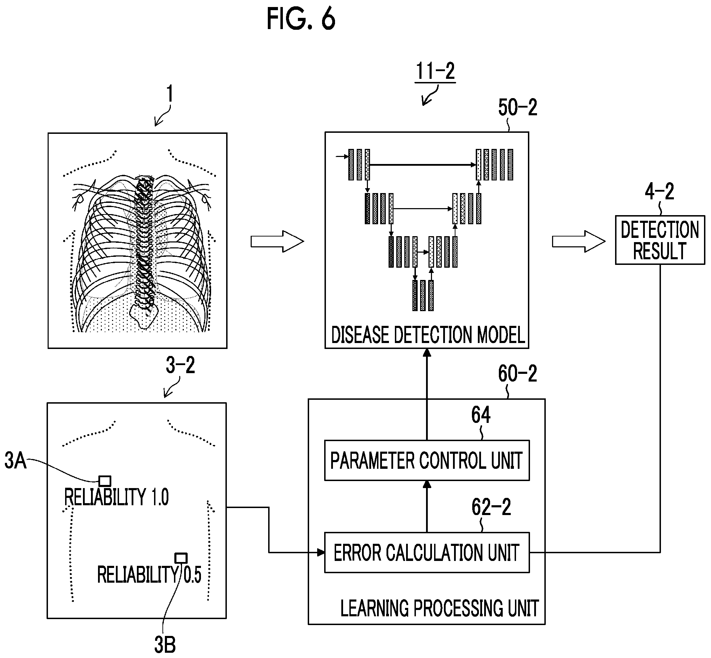

The processor 12 is composed of a central processing unit (CPU) and the like and controls an overall operation of each unit of the disease label creation device 10. For example, the processor 12 functions as an information acquisition unit 28, a registration processing unit 30, a disease label converter 40, and a reliability calculator 42 in disease label creation devices 10-1 and 10-2 illustrated in Figs. 2 and 4. In addition, the processor 12 functions disease detection models 50-1 and 50-2 and learning processing units 60-1 and 60-2 in learning devices 11-1 and 11-2 illustrated in Figs. 5 and 6.

The memory 14 includes a flash memory, a read-only memory (ROM), a random access memory (RAM), a hard disk apparatus, and the like. The flash memory, the ROM, and the hard disk apparatus are non-volatile memories that store, for example, various programs including an operation system, a program and parameters for causing the processor 12 to function as the disease detection model, and a disease label creation program according to the present invention.

The RAM functions as a work area for processing by the processor 12 and temporarily stores, for example, the disease label creation program stored in the non-volatile memory. However, a portion (RAM) of the memory 14 may be provided in the processor 12.

The first database 16 is a portion that stores and manages simple X-ray images of a large number of patients, three-dimensional CT images (same patients) paired with each simple X-ray image, and a first disease label indicating three-dimensional region information of a disease (information related to, for example, three-dimensional coordinates of the disease) extracted from each CT image. Further, a class classification of the disease may be added as an annotation to the first disease label. In addition, a segmentation label indicating a region of each of a thorax, a lung field, and other organs may be attached to the CT image.

Furthermore, the simple X-ray image according to this example is a chest X-ray image, and the CT image is obtained by capturing slice images of a chest in a cross-sectional direction while changing a position in a body axis direction. The three-dimensional CT image is three-dimensional data obtained by superimposing a large number of slice images obtained by imaging in the cross-sectional direction in the body axis direction.

The second database 18 is a portion that stores and manages training data consisting of a pair of the simple X-ray image and the disease label (second disease label) created by the disease label creation device 10. In a case in which the second disease label is created on the basis of the simple X-ray image and the CT image paired with each simple X-ray image stored in the first database 16, the second database 18 is a storage destination of training data that consists of a pair of the simple X-ray image and the created second disease label.

In addition, the first database 16 and the second database 18 may be physically the same. Further, at least one of the first database 16 or the second database 18 may be provided outside the device and may exchange data through the input/output interface 22.

The display 20 is a portion that displays, for example, the simple X-ray image and a detection result of a disease detected by a trained disease detection model in a case in which the disease is detected from the simple X-ray image to be diagnosed. In a case in which a user (doctor) observes the simple X-ray image to be diagnosed and performs image diagnosis, the user (doctor) can perform the diagnosis with reference to the detection result and the like displayed on the display 20. Further, the display 20 can be used as a portion of a user interface in a case in which various instructions are received from the user.

The input/output interface 22 includes, for example, a connection unit that can be connected to an external apparatus and a communication unit that can be connected to a network. For example, a universal serial bus (USB) or a high-definition multimedia interface (HDMI) (HDMI is a registered trademark) can be applied as the connection unit that can be connected to the external apparatus. The processor 12 can acquire various programs including the disease label creation program stored in the memory 14 and the information (for example, the simple X-ray images and the CT images for creating the training data) stored in the first database 16 through the input/output interface 22, in addition to the simple X-ray image to be diagnosed. In addition, it is possible to use an external display device connected to the input/output interface 22 instead of the display 20.

The operation unit 24 includes a keyboard, a pointing device, such as a mouse, and a keyboard and functions as a user interface that receives various instructions by the doctor.

### [First Embodiment of Disease Label Creation Device]

Fig. 2 is a functional block diagram illustrating a first embodiment of the disease label creation device according to the present invention.

The disease label creation device 10-1 according to the first embodiment illustrated in Fig. 2 is configured by the processor 12 (first processor) and the memory 14 included in the disease label creation device 10 having the hardware configuration illustrated in Fig. 1. As described above, the processor 12 functions as the information acquisition unit 28, the registration processing unit 30, and the disease label converter 40.

The information acquisition unit 28 is a portion that performs an information acquisition process of acquiring information, such as a simple X-ray image 1 and a three-dimensional CT image 2 (same patient) with a disease label which is paired with the simple X-ray image 1, from the first database 16.

The disease label (first disease label) is three-dimensional region information indicating a three-dimensional disease region in the CT image 2 and is information of each pixel of the three-dimensional disease region or information indicating a rectangular parallelepiped region surrounding the three-dimensional disease region.

The first disease label can be automatically (easily) acquired by inputting a plurality of slice images (axial images) constituting the three-dimensional CT image 2 one by one to a well-known disease detection model and integrating the detection results. In addition, in the slice images of the CT image, since the contrast between a normal region and a disease region is clear, it is possible to extract the disease region with high accuracy. Further, in this example, the first disease label extracted in advance is attached to the three-dimensional CT image 2. However, in a case in which the first disease label is not attached, the first disease label may be automatically detected on the basis of the three-dimensional CT image.

The registration processing unit 30 receives the pair of the simple X-ray image 1 and the CT image 2 acquired by the information acquisition unit 28 as an input and performs registration between the simple X-ray image 1 and the CT image 2. A registration process of the registration processing unit 30 can be performed by, for example, projecting the three-dimensional CT image 2 to be converted into a two-dimensional DRR image and adjusting geometric conditions in the projection, the rotation of the CT image 2, and the like such that the converted DDR image is matched with the simple X-ray image 1. Further, here, an example in which the registration is performed by rigid deformation has been described. However, the registration may be performed by non-rigid deformation. In addition, the registration processing unit 30 will be described in detail below.

The disease label converter 40 performs a conversion process of, for example, projecting a three-dimensional first disease label on the basis of the result of the registration by the registration processing unit 30 to convert the three-dimensional first disease label into a two-dimensional disease label (second disease label) 3-1 corresponding to the simple X-ray image 1 in the same manner as the three-dimensional CT image 2.

In a case in which information indicating regions of rectangular parallelepipeds 2A and 2B surrounding two three-dimensional disease regions (for example, coordinate information of eight vertices of each rectangular parallelepiped) is used as the three-dimensional first disease label, three-dimensional coordinate information of the eight vertices of each rectangular parallelepiped is converted into two-dimensional coordinate information indicating eight positions on the simple X-ray image 1 by the disease label converter 40. Then, information indicating rectangular regions 3A and 3B including eight two-dimensional coordinate information items can be used as the disease label 3-1 for the simple X-ray image 1.

In a case in which the disease label creation device 10-1 creates the disease label 3-1 for the simple X-ray image 1, the processor 12 stores a pair of the simple X-ray image 1 and the disease label 3-1 as training data (first training data) in the second database 18.

In addition, the disease label 3-1 according to this example is region information in a rectangular frame surrounding the disease region in the simple X-ray image 1, but may be coordinate information of two diagonal vertices of the rectangular frame (bounding box). Moreover, the first disease label in the CT image 2 may be region information indicating the three-dimensional disease region. In this case, the two-dimensional disease label is information of the disease region (each pixel) in the simple X-ray image 1.

Further, in a case in which the first disease label in the CT image 2 is region information indicating the three-dimensional disease region, the disease label converter 40 can project the first disease label to convert the first disease label into a pixel-level disease region in the simple X-ray image 1 and further convert the bounding box circumscribing the pixel-level disease region into the disease label 3-1 (second disease label).

Furthermore, in a case in which the class classification of the disease (a size and volume; in the case of a lung disease, a lung nodule, a lung tumor, and the like) is given as an annotation to the three-dimensional first disease label, it is preferable to also give a disease class classification annotation to the converted disease label 3-1 on a disease basis or an image basis.

### [Registration Processing Unit]

### <First Embodiment of Registration Processing Unit>

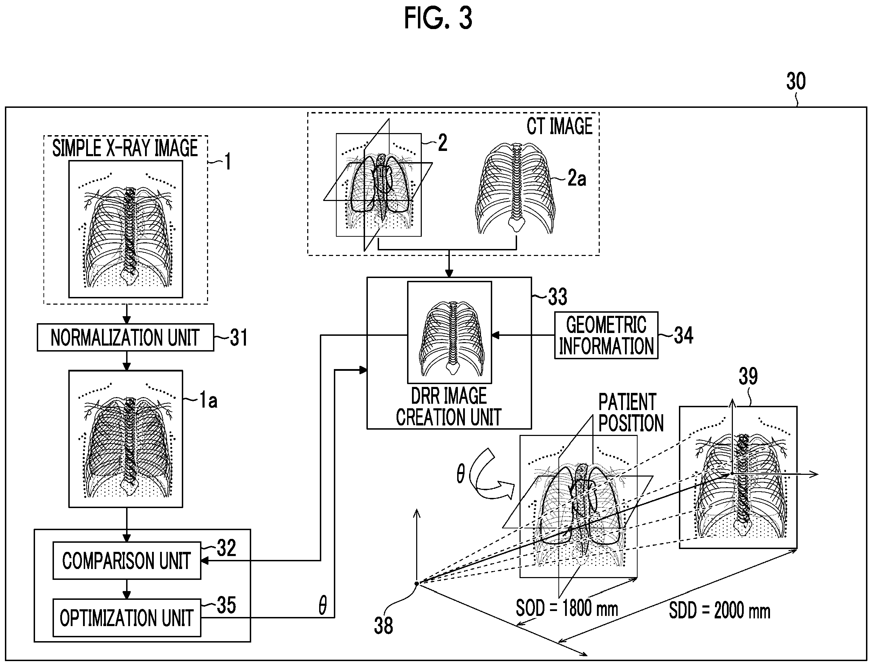

Fig. 3 is a block diagram illustrating a first embodiment of the registration processing unit illustrated in Fig. 2.

The registration processing unit 30 illustrated in Fig. 3 comprises a normalization unit 31, a comparison unit 32, a DRR image creation unit 33, and an optimization unit 35.

In a case in which the simple X-ray image 1 is input, for example, the normalization unit 31 normalizes a gray value for each local region of the simple X-ray image 1 to adjust contrast. A normalized simple X-ray image 1a is added to the comparison unit 32.

The three-dimensional CT image 2 and mask information 2a indicating a specific region of the three-dimensional CT image 2 are added to the DRR image creation unit 33. First, the DRR image creation unit 33 extracts the specific region of the CT image 2 on the basis of the mask information 2a. The specific region according to this example is a thorax region which is a bone region, and the thorax region is extracted from the CT image 2 on the basis of the mask information 2a indicating the thorax region. In a case in which the mask information 2a is given as information indicating each organ region of the CT image 2, the information can be used. In a case in which the mask information 2a is not given, the thorax region may be directly extracted from the CT image 2.

Geometric information in a case in which the CT image 2 (thorax region) is projected to be converted into a two-dimensional DRR image is added to another input of the DRR image creation unit 33, and the DRR image creation unit 33 projects the thorax region on the basis of the geometric information 34 to create a DRR image which is a two-dimensional image of the thorax region.

Here, the geometric information 34 is geometric information in a case in which the CT image 2 is projected onto a projection plane and includes, for example, a distance (SOD) between a X-ray source position 38 and a patient position (projected CT image) and a distance (SDD) between the X-ray source position 38 and a detector position 39. The distance (SOD) and the distance (SDD) can be acquired from, for example, a positional relationship between an X-ray source and a detector (imaging plate) in a case in which the simple X-ray image 1 is captured. Further, the geometric information 34 can be used as a parameter for adjusting, for example, the size of the DRR image.

The DRR image created by the DRR image creation unit 33 is added to the comparison unit 32.

The comparison unit 32 compares density gradients of the normalized simple X-ray image 1a and the DRR image and outputs a comparison result indicating a similarity between the two images to the optimization unit 35.

The optimization unit 35 outputs, to the DRR image creation unit 33, information for adjusting a posture parameter (θ) of the projected CT image 2 (thorax region) on the basis of the comparison result output from the comparison unit 32 such that the similarity is maximized. Further, the optimization unit 35 can output information for adjusting the geometric information 34.

The DRR image creation unit 33 outputs, to the comparison unit 32, the DRR image obtained by adjusting projection conditions (the posture parameter (θ) and the geometric information 34) on the basis of the information input from the optimization unit 35.

By sequentially repeating the processes of the comparison unit 32, the optimization unit 35, and the DRR image creation unit 33, the DRR image creation unit 33 creates the DRR image having the maximum similarity with the normalized simple X-ray image 1a.

Then, the registration processing unit 30 can output the projection conditions, in which the similarity between the simple X-ray image 1a and the DRR image is maximized, as the result of the registration to the disease label converter 40. The disease label converter 40 projects the three-dimensional first disease label on the basis of the result of the registration (projection conditions) to convert the three-dimensional first disease label into the two-dimensional disease label 3-1 corresponding to the simple X-ray image.

In this example, the registration with the simple X-ray image 1 is performed using the thorax region of the CT image 2 to register a lung field region in the thorax region. However, the present invention is not limited thereto. For example, the registration with the simple X-ray image 1 may be performed using the lung field region of the CT image 2. Alternatively, the registration with the simple X-ray image 1 may be performed using a plurality of organ regions.

In addition, the registration processing unit 30 may execute a process of projecting the CT image 2 to create a pseudo X-ray image and performing registration between the simple X-ray image 1 and the pseudo X-ray image.

Further, the disease label converter 40 illustrated in Fig. 2 can two-dimensionally convert the first disease label of the registered CT image 2 (projects the first disease label onto the same plane as the simple X-ray image 1) into the second disease label corresponding to the simple X-ray image 1.

### <Second Embodiment of Registration Processing Unit>

A second embodiment of the registration processing unit 30 illustrated in Fig. 3 performs the registration using a two-dimensional anatomical landmark of the simple X-ray image 1 and a three-dimensional anatomical landmark of the CT image 2 corresponding to the two-dimensional anatomical landmark.

That is, the registration processing unit 30 according to the second embodiment performs a process of extracting the two-dimensional anatomical landmark from the simple X-ray image 1 and performs a process of extracting the three-dimensional anatomical landmark (a landmark corresponding to the two-dimensional anatomical landmark) from the CT image 2. For example, each rib or a boundary of each rib with costal cartilage can be used as the anatomical landmark.

The registration processing unit 30 performs a process of projecting the extracted three-dimensional anatomical landmark and performs a process of performing registration between the two-dimensional anatomical landmark and the anatomical landmark after the projection process. That is, the projection conditions of the three-dimensional anatomical landmark are adjusted such that the two anatomical landmarks are matched with each other, and the projection is performed.

The registration processing unit 30 outputs, as the result of the registration, the projection condition in which the two-dimensional anatomical landmark and the projected three-dimensional anatomical landmark are matched with each other or in which the ratio of match is the highest value.

### <Third Embodiment of Registration Processing Unit>

A third embodiment of the registration processing unit 30 illustrated in Fig. 3 performs the registration using a two-dimensional anatomical region of interest of the simple X-ray image 1 and a three-dimensional anatomical region of interest of the CT image 2 corresponding to the two-dimensional anatomical region of interest.

That is, the registration processing unit 30 according to the third embodiment performs a process of extracting the two-dimensional anatomical region of interest from the simple X-ray image 1 and performs a process of extracting the three-dimensional anatomical region of interest (a region of interest corresponding to the two-dimensional anatomical region of interest) from the CT image 2. Examples of the anatomical region of interest in the simple X-ray image 1 of the chest include regions of a lung field, a thorax, a heart, and an aorta.

The registration processing unit 30 performs a process of projecting the extracted three-dimensional anatomical region of interest and performs registration between a contour of the two-dimensional anatomical region of interest and a contour of the anatomical region of interest after the projection process. That is, the projection conditions of the three-dimensional anatomical region of interest are adjusted such that the contours of the two anatomical regions of interest are matched with each other, and the projection is performed.

The registration processing unit 30 outputs, as a result of the registration, the projection condition in which the contour of the two-dimensional anatomical region of interest and the contour of the projected three-dimensional anatomical region of interest are matched with each other or the ratio of match is the highest value.

### <Fourth Embodiment of Registration Processing Unit>

A fourth embodiment of the registration processing unit 30 illustrated in Fig. 3 performs the registration using a three-dimensionally restored simple X-ray image and a three-dimensional CT image.

That is, the registration processing unit 30 according to the fourth embodiment performs a process of three-dimensionally restoring the simple X-ray image 1. For example, a learning model that has been trained to output a three-dimensional X-ray image in a case in which a two-dimensional simple X-ray image is input can be used to three-dimensionally restore the simple X-ray image 1.

The registration processing unit 30 performs a process of performing registration between the three-dimensional CT image 2 and the three-dimensionally restored simple X-ray image. The registration in this case is registration in a three-dimensional space. For example, the CT image 2 is registered with the three-dimensionally restored simple X-ray image by the translational movement, rotational movement, enlargement and reduction, and the like of the CT image 2.

### <Fifth Embodiment of Registration Processing Unit>

A fifth embodiment of the registration processing unit 30 performs the registration by adjusting a solution space in the registration between the simple X-ray image and the CT image forming the pair associated with a patient, depending on the patient.

For example, the disease label creation device 10 comprises a database of a statistical deformation model for each patient feature information item. The registration processing unit 30 according to the fifth embodiment selects a corresponding statistical deformation model from the database of the statistical deformation model on the basis of the patient feature information of a patient corresponding to the pair of the simple X-ray image 1 and the CT image 2.

The registration processing unit 30 performs non-rigid registration between the simple X-ray image 1 and the CT image 2 using the selected statistical deformation model.

For a patient with a disorder in which the ribs do not move, in a case in which the positions of the ribs in the simple X-ray image and the CT image of the patient are registered, a statistical deformation model corresponding to the patient (disorder) is selected, and the non-rigid registration between the simple X-ray image and the CT image is performed using the selected statistical deformation model.

### [Second Embodiment of Disease Label Creation Device]

Fig. 4 is a functional block diagram illustrating a second embodiment of the disease label creation device according to the present invention. In addition, in Fig. 4, portions common to the disease label creation device 10-1 according to the first embodiment illustrated in Fig. 2 are denoted by the same reference numerals, and the detailed description thereof will not be repeated.

A disease label creation device 10-2 according to the second embodiment illustrated in Fig. 4 is different from the disease label creation device 10-1 according to the first embodiment in that a reliability calculator 42 is added.

The reliability calculator 42 performs a first reliability calculation process of calculating a reliability (first reliability) for the second disease label converted by the disease label converter 40. The calculated first reliability is given as an annotation of the created second disease label to the second disease label. That is, a disease label 3-2 is different from the disease label 3-1 to which the reliability is not given in that it is a disease label with a reliability.

### <First Embodiment of Reliability Calculator>

A first embodiment of the reliability calculator 42 illustrated in Fig. 4 calculates the reliability (first reliability) of a disease region (second disease region) in the simple X-ray image 1 from the visibility of the disease region.

The reliability calculator 42 according to the first embodiment calculates the visibility of the second disease region corresponding to the second disease label with respect to a normal region of the simple X-ray image 1, using at least one of the statistics of the pixel values of the first disease region of the CT image 2 corresponding to the three-dimensional first disease label or the shape feature, and calculates the first reliability for the second disease label from the calculated visibility.

For example, in a case in which the first reliability has a value in the range of 0 to 1.0, the first reliability is calculated such that the first reliability is closer to 0 as the visibility is lower and is closer to 1.0 as the visibility is higher.

The visibility of the second disease region can be evaluated by the statistics (for example, an average value or a density variance) of the pixel values of the normal region and the first disease region. The reason is that, in a case in which the difference between the statistics of the pixel values of the normal region and the first disease region is large, it is easy to distinguish between the normal region and the second disease region (a disease region of the simple X-ray image 1 corresponding to the first disease region) in the simple X-ray image 1; and, in a case in which the difference is small, it is difficult to distinguish between the normal region and the second disease region.

In addition, the visibility of the second disease region can be evaluated by the shape feature of the first disease region. For example, the reason is that, as the size of the first disease region is smaller, the size of the second disease region corresponding to the first disease region is smaller and it is more difficult to find the second disease region. Further, it is preferable that the size of the first disease region is a size in a case in which the first disease region is projected.

Moreover, the visibility of the second disease region can be evaluated by the pixel value and shape feature of the first disease region. For example, an X-ray absorption amount is proportional to the product of the thickness of a region in a projection direction and a pixel value (linear attenuation coefficient) of the region. The reason is that, in a case in which the X-ray absorption amount of the disease region with respect to the normal region is large, it is easy to distinguish between the normal region and the second disease region in the simple X-ray image 1; and, in a case in which the X-ray absorption amount of the disease region is small, it is difficult to distinguish between the normal region and the second disease region. Further, the shape feature is not limited to the shape feature in the projection direction and may be a shape feature related to the entire disease region.

### <Second Embodiment of Reliability Calculator>

In a second embodiment of the reliability calculator 42 illustrated in Fig. 4, the information acquisition unit 28 acquires information of an anatomical region in the CT image 2 from the first database 16 or through the input/output interface 22. The information of the anatomical region is, for example, three-dimensional region information for each organ, such as the thorax, the lung field, and the heart.

The reliability calculator 42 according to the second embodiment calculates the visibility of the second disease region corresponding to the second disease label with respect to the normal region of the simple X-ray image 1 on the basis of the superimposition of the anatomical region and the first disease region of the CT image 2 corresponding to the first disease label in the projection direction.

In a case in which the first disease region of the CT image 2 is superimposed on the anatomical region (for example, a thorax region which is a bone region) in the projection direction, the X-ray transmission amount of the disease region (second disease region) of the simple X-ray image 1 corresponding to the first disease region is reduced due to the bone region, and the visibility of the disease region is reduced. In a case in which the first disease region and the anatomical region (particularly, the bone region) are superimposed in the projection direction, the reliability calculator 42 reduces the visibility of the second disease region as compared to a case in which the first disease region and the anatomical region are not superimposed.

In addition, in a case in which the first disease region and the anatomical region are superimposed in the projection direction, it is preferable to calculate the visibility according to a degree of superimposition (partial superimposition or complete superimposition) and the type of the superimposed anatomical region.

The reliability calculator 42 according to the second embodiment calculates the first reliability from the visibility calculated according to the superimposition on the anatomical region as described above.

### <Third Embodiment of Reliability Calculator>

In a third embodiment of the reliability calculator 42 illustrated in Fig. 4, the information acquisition unit 28 acquires an interpretation report corresponding to the simple X-ray image 1 or the CT image 2 from the first database 16 or through the input/output interface 22. The results (for example, a disease part, a class classification of a disease, and a size) of the image diagnosis using the simple X-ray image 1 or the CT image 2 are described in the interpretation report.

The reliability calculator 42 according to the third embodiment calculates the first reliability for the first disease label which has been automatically detected, on the basis of the rate of match between the disease label (first disease label) automatically detected from the CT image 2 and content (content of the results of the image diagnosis) described in the interpretation report.

This makes it possible to reflect the results of the image diagnosis by the specialized doctor in the first reliability.

### <Fourth Embodiment of Reliability Calculator>

The processor 12 or the registration processing unit 30 calculates a degree of success of the result of the registration between the simple X-ray image 1 and the CT image 2.

For the degree of success of the registration, for example, in a case in which the registration is performed on the basis of a plurality of anatomical landmarks detected from the simple X-ray image 1 and a plurality of anatomical landmarks detected from the CT image 2 which correspond to the plurality of anatomical landmarks and are projected onto the projection plane, the magnitude of the square sum of the distances between the corresponding anatomical landmarks can be used as an indicator of the degree of success.

The fourth embodiment of the reliability calculator 42 illustrated in Fig. 4 calculates the first reliability for the second disease label on the basis of the degree of success of the registration between the simple X-ray image 1 and the CT image 2.

### <Fifth Embodiment of Reliability Calculator>

A fifth embodiment of the reliability calculator 42 illustrated in Fig. 4 reduces the first reliability for the second disease label of a region having different imaging ranges (visual fields) in the simple X-ray image 1 and the CT image 2.

For example, it is considered that, in a case in which the imaging range of the CT image is narrower than the imaging range of the simple X-ray image, the processor 12 extrapolates the CT image and performs registration between the simple X-ray image and the extrapolated CT image. Meanwhile, it is considered that the first disease label is automatically detected from the extrapolated CT image.

In a case in which the automatically detected first disease label is included in an extrapolated region of the extrapolated CT image, the reliability calculator 42 according to the fifth embodiment gives a low first reliability to the second disease label corresponding to the first disease label. That is, the first reliability for the second disease label is reduced such that the second disease label is not treated as the disease label.

In addition, the reliability calculator 42 can integrate two or more first reliabilities calculated by two or more of the first to fifth embodiments to obtain a new first reliability.

In a case in which the disease label creation device 10-2 creates the disease label 3-2 with a reliability for the simple X-ray image 1, the processor 12 stores the simple X-ray image 1, the second disease label, and the first reliability (disease label 3-2 with a reliability) as second training data in the second database 18.

In addition, the information acquisition unit 28 can acquire an image-level disease label (third disease label) of the CT image 2. The image-level third disease label includes the presence or absence of a disease, a class classification (size or volume) of a disease, the number of diseases, the presence or absence of a disease in each of the right and left lungs, and the like.

In a case in which the image-level third disease label is given as the annotation of the CT image 2, the information acquisition unit 28 can acquire the given third disease label. In a case in which the third disease label is not given, the information acquisition unit 28 can acquire an image-level third disease label obtained by converting a pixel-level first disease label for the CT image 2 with a converter (not illustrated).

In a case in which the image-level third disease label is acquired, the processor 12 gives the first disease label and the third disease label to the simple X-ray image 1 and stores them as the training data in the second database.

In addition, the processor 12 or the registration processing unit 30 can calculate the degree of success of the result of the registration between the simple X-ray image 1 and the CT image 2 as described above. The processor 12 determines whether the result of the registration is a success or a failure on the basis of the degree of success of the result of the registration.

The processor 12 selects the second disease label in a case in which it is determined that the result is a success, selects the third disease label in a case in which it is determined that the result is a failure, and gives the selected second or third disease label to the simple X-ray image.

That is, in a case in which the registration between the simple X-ray image 1 and the CT image 2 has failed, a pair of the simple X-ray image and the image-level third disease label is used as the training data (third training data), and the second training data which is noise is excluded.

### [First Embodiment of Learning Device]

Fig. 5 is a functional block diagram illustrating a first embodiment of the learning device according to the present invention.

A learning device 11-1 according to the first embodiment illustrated in Fig. 5 is configured by the processor 12 (second processor) and the memory 14 included in the disease label creation device 10 having the hardware configuration illustrated in Fig. 1, and the processor 12 functions as a disease detection model 50-1 and a learning processing unit 60-1 as described above.

The learning device 11-1 trains the disease detection model 50-1, using the first training data (learning data set) consisting of the simple X-ray image and the second disease label stored in the second database 18.

The disease detection model 50-1 is a learning model that receives the simple X-ray image as an input, detects a disease label from the input simple X-ray image, and outputs the disease label is preferably a learning model consisting of a Bayesian neural network and is more preferably Bayesian U-Net. In addition, in the Bayesian neural network, desired learning can be performed even with a small number of data sets. Therefore, it is possible to further reduce an annotation cost for preparing the data sets.

The disease detection model 50-1 before training by the device may be a model that has not been trained or may be, for example, a model that has been trained to attach a label (segmentation label) to each region of the image.

The processor 12 reads out the first training data consisting of the simple X-ray image 1 and the disease label 3-1 (second disease label) stored in the second database 18, inputs the simple X-ray image 1 to the disease detection model 50-1, and outputs the disease label 3-1 as a correct answer label to the learning processing unit 60-1.

The disease detection model 50-1 that receives the simple X-ray image 1 as an input has a plurality of layer structures, such as a convolutional layer and a pooling layer, and holds parameters such as a coefficient of a filter applied to the convolutional layer, an offset value, and a weight for connection between the preceding and succeeding layers. The disease detection model 50-1 functions as a trained disease detection model that detects a disease by setting the parameters to the optimum values. The disease detection model 50-1 according to this example is a model that performs segmentation to individually recognize a disease region, such as a lung nodule (oval shadow), included in the simple X-ray image 1, performs region classification (segmentation) of each disease region for each pixels in the simple X-ray image 1 or for each group consisting of several pixels, and outputs, for example, a label image indicating each disease region or a bounding box surrounding the disease region as a detection result 4-1 for each disease region. In addition, in the disease detection model 50-1 that has not been trained, the parameters are not set to the optimum values, and it is not possible to output the appropriate detection result 4-1.

The learning processing unit 60-1 comprises an error calculation unit 62-1 and a parameter control unit 64.

The disease label 3-1 is added to one input of the error calculation unit 62-1, and the detection result 4-1 which is the output of the disease detection model 50-1 is added to another input. The error calculation unit 62-1 calculates an error (first error) between the disease label 3-1 and the detection result 4-1. For example, softmax cross entropy or sigmoid is considered as a method for calculating the error.

The parameter control unit 64 adjusts the parameters in the disease detection model 50-1 using a backpropagation method on the basis of the first error calculated by the error calculation unit 62-1 to train the disease detection model 50-1.

The learning processing unit 60-1 repeatedly adjusts the parameters in the disease detection model 50-1 such that the first error converges. This is performed using a large number of first training data items (learning data sets) stored in the second database 18 to train the disease detection model 50-1.

Further, the learning processing unit 60-1 may train the disease detection model 50-1 using the training data (third training data) consisting of the simple X-ray image and the image-level disease label (third disease label) of the CT image corresponding to the simple X-ray image, in addition to training the disease detection model 50-1 using the first training data.

That is, the error calculation unit 62-1 calculates an integrated error of the first error between the disease label 3-1 and the detection result 4-1 and the second error between the image-level third disease label of the CT image and the detection result (image-level disease detection result), and the parameter control unit 64 adjusts the parameters in the disease detection model 50-1 using the backpropagation method on the basis of the integrated error to train the disease detection model 50-2.

The disease detection model 50-1 trained in this way can output the image-level disease label in addition to, for example, the pixel-level disease label.

In addition, the learning processing unit 60-1 does not train the disease detection model 50-1 with each first training data item, but can extract a predetermined number of mini-batches of first training data from the second database 18, train the disease detection model 50-1 such that the total first error of each mini-batch converges, and perform this process for the plurality of mini-batches to train the disease detection model 50-1. In this case, the first training data and the third training data are mixed in the second database 18, which makes it possible to include the first training data and the third training data in the mini-batch. Therefore, it is possible to train the disease detection model 50-1 on the basis of the error of each mini-batch (the integrated error of the first error and the second error) such that the error converges.

### [Second Embodiment of Learning Device]

Fig. 6 is a functional block diagram illustrating a second embodiment of the learning device according to the present invention.

A learning device 11-2 according to the second embodiment illustrated in Fig. 6 is configured by the processor 12 (second processor) and the memory 14 included in the disease label creation device 10 having the hardware configuration illustrated in Fig. 1. As described above, the processor 12 functions as a disease detection model 50-2 and a learning processing unit 60-2.

The learning device 11-2 according to the second embodiment is different from the learning device 11-1 according to the first embodiment illustrated in Fig. 5 which performs training using the training data (first training data) consisting of the simple X-ray image 1 and the disease label 3-1 to which the reliability is not given in that the learning device 11-2 performs training using training data (second training data) consisting of the simple X-ray image 1, the reliability (first reliability) for the second disease label, and the second disease label (disease label with a reliability) 3-2.

The disease detection model 50-2 is a learning model that receives the simple X-ray image as an input, detects a disease label from the input simple X-ray image, and outputs the disease label is preferably a learning model consisting of a Bayesian neural network, and is more preferably Bayesian U-Net.

The processor 12 reads out the second training data consisting of the simple X-ray image 1 and the disease label 3-2 with a reliability stored in the second database 18, inputs the simple X-ray image 1 to the disease detection model 50-2, and outputs the disease label 3-2 to the learning processing unit 60-1.

The disease detection model 50-2 that receives the simple X-ray image 1 as an input functions as a trained disease detection model that detects a disease by setting the parameters to the optimum values, similarly to the disease detection model 50-1 illustrated in Fig. 5. That is, the disease detection model 50-2 is a model that performs segmentation to individually recognize a disease region, such as a lung nodule, included in the simple X-ray image 1, performs region classification (segmentation) of each disease region for each pixel in the simple X-ray image 1 or for each group consisting of several pixels, and outputs, for example, a label image indicating each disease region or a bounding box surrounding the disease region as a detection result 4-2 for each disease region. In addition, in the disease detection model 50-2 that has not been trained, the parameters are not set to the optimum values, and it is not possible to output the appropriate detection result 4-2.

The learning processing unit 60-2 comprises an error calculation unit 62-2 and a parameter control unit 64.

The disease label 3-2 with a reliability is added to one input of the error calculation unit 62-2, and the detection result 4-2 which is the output of the disease detection model 50-2 is added to the other input. The error calculation unit 62-2 calculates an error (first error) between the disease label 3-2 and the detection result 4-2 and adjusts the calculated first error according to the first reliability attached to the disease label 3-2. For example, in a case in which the first reliability has a value in the range of 0 to 1.0, the first error can be adjusted by multiplying the first error by the first reliability.

The parameter control unit 64 adjusts the parameters in the disease detection model 50-2 using the backpropagation method on the basis of the first error, which has been calculated by the error calculation unit 62-2 and adjusted by the first reliability, to train the disease detection model 50-2.

The learning processing unit 60-2 repeatedly adjusts the parameters in the disease detection model 50-2 such that the first error converges. This is performed using a large number of second training data items (learning data sets) stored in the second database 18 to train the disease detection model 50-2.

In addition, the learning processing unit 60-2 does not train the disease detection model 50-2 with each second training data item, but can extract a predetermined number of mini-batches of second training data from the second database 18, train the disease detection model 50-1 such that the total first error of each mini-batch (the total error of the first errors adjusted by the first reliability of each second training data item of the mini-batch) converges, and perform this process for the plurality of mini-batches to train the disease detection model 50-2.

In a case in which an annotation at a level that is visually recognized in the CT image, but is not visually recognized or is difficult to visually recognize in the simple X-ray image is reflected in learning, it may be noise in the training of the model. However, according to the learning device 11-2 according to the second embodiment, the first error is adjusted according to the first reliability, and the model is trained. Therefore, it is possible to reduce the influence of the annotation noise.

In a modification example of the learning device 11-2 according to the second embodiment, the disease detection model 50-2 is trained to output the reliability (second reliability) as the detection result. Therefore, the disease detection model 50-2 can be a learning model that, in a case in which the simple X-ray image is input, detects a disease label and the reliability (second reliability) of the disease label detected by the disease detection model 50-2 (that is, a disease label with a second reliability) from the input simple X-ray image and outputs them.

In this case, the error calculation unit 62-2 calculates the first error between the disease label 3-2 with the first reliability and the detection result 4-2 with the second reliability and adjusts the calculated first error A according to the first reliability attached to the disease label 3-2.

Further, the error calculation unit may integrate the first reliability and the second reliability to generate a third reliability and may adjust the first error according to the third reliability in a case in which a learning process of converging the first error is performed.

Furthermore, it is preferable that the error calculation unit 62-2 adjusts the first error of a disease region, of which has the second reliability output from the disease detection model 50-2 is low and which is false positive, to a large value and adjusts the first error of a disease region, of which the second reliability is low and which is false negative, to a small value. The reason is that, in a case in which the second reliability is low, training is performed such that the detection result of a false positive is not output.

The parameter control unit 64 adjusts the parameters in the disease detection model 50-2 using the backpropagation method on the basis of the first error, which has been calculated by the error calculation unit 62-2 and adjusted by the first reliability, to train the disease detection model 50-2.

### [First Embodiment of Disease Detection Model]

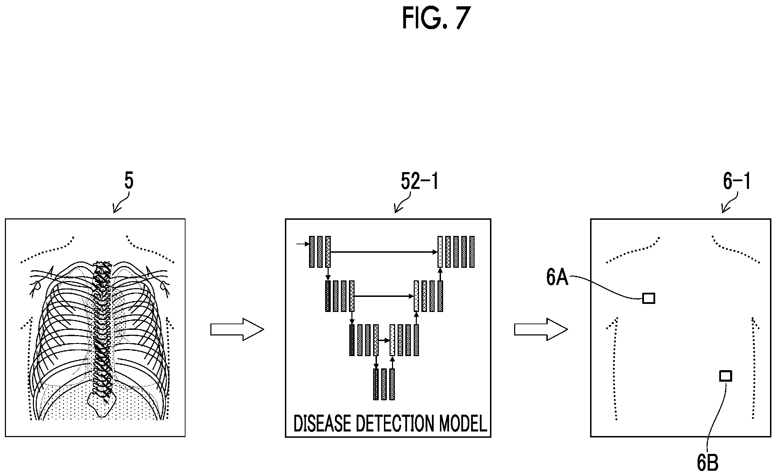

Fig. 7 is a diagram illustrating a first embodiment of the disease detection model according to the present invention and particularly illustrates the input and output of the disease detection model.

A disease detection model 52-1 according to the first embodiment illustrated in Fig. 7 is a model that corresponds to the trained disease detection model 50-1 trained by the learning device 11-1 according to the first embodiment. The disease detection model 52-1 may be the trained disease detection model 50-1 trained by the learning device 11-1 or may be another disease detection model in which the parameters optimized in the trained disease detection model 50-1 have been set.

The disease detection model 52-1 receives, as an input image, any simple X-ray image 5 other than the simple X-ray image used during learning and outputs a disease label detected from the input simple X-ray image 5 as a detection result 6-1.

In the example illustrated in Fig. 7, the disease detection model 52-1 detects, as the disease regions, lung nodules 6A and 6B which are oval shadows and outputs bounding boxes surrounding the lung nodules 6A and 6B (bounding boxes with annotations for class classification of the lung nodules) as the detection result 6-1.

The simple X-ray image 5 is displayed on the display 20 during image diagnosis by the specialized doctor. The bounding boxes surrounding the lung nodules 6A and 6B, which are the detection results of the disease detection model 52-1, can be displayed to be superimposed on the simple X-ray image 5 displayed on the display 20, which makes it possible to support the image diagnosis by the specialized doctor. In addition, the class classification of the lung nodule may be displayed by text or may be displayed by the color of a frame of the bounding box.

In addition, the display and non-display of the bounding box can be selected. Further, the bounding box is an example of the indicator indicating the disease region. Instead of using the bounding box, the contour of the disease region may be highlighted, or the disease region may be indicated by, for example, an arrow.

### [Second Embodiment of Disease Detection Model]

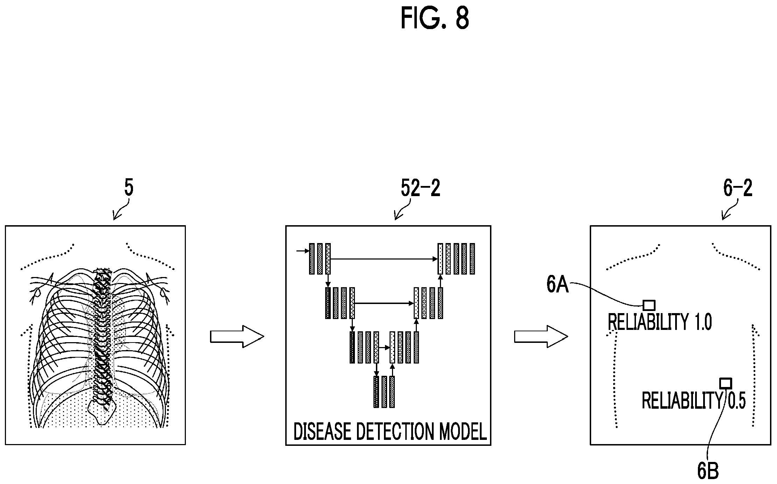

Fig. 8 is a diagram illustrating a second embodiment of the disease detection model according to the present invention and particularly illustrates the input and output of the disease detection model.

A disease detection model 52-2 according to the second embodiment illustrated in Fig. 8 corresponds to the trained disease detection model 50-2 trained by the learning device 11-2 according to the second embodiment and is particularly a model that has been trained to output a detection label with a reliability.

The disease detection model 52-2 receives, as an input image, any simple X-ray image 5 other than the simple X-ray image used during learning and outputs the disease label with a reliability detected from the input simple X-ray image 5 as a detection result 6-2.

In the example illustrated in Fig. 8, the disease detection model 52-2 detects the lung nodules 6A and 6B, which are oval shadows, and the reliability of each of the lung nodules 6A and 6B and outputs the bounding boxes surrounding the lung nodules 6A and 6B and numerical values (1.0 and 0.5) indicating the reliabilities as the detection result 6-2.

The simple X-ray image 5 is displayed on the display 20 during image diagnosis by the specialized doctor. The bounding boxes with the reliabilities surrounding the lung nodules 6A and 6B, which are the detection results of the disease detection model 52-2, can be displayed to be superimposed on the simple X-ray image 5 displayed on the display 20, which makes it possible to support the image diagnosis by the specialized doctor. In addition, the bounding boxes with the reliabilities make it possible to understand the visibility of the diseases (lung nodules 6A and 6B) surrounded by the bounding boxes.

### [Disease Label Creation Method]

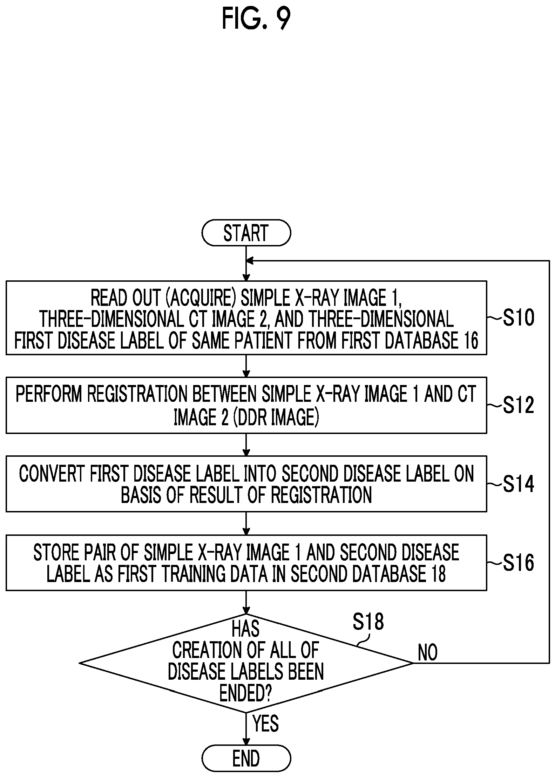

Fig. 9 is a flowchart illustrating an embodiment of a disease label creation method according to the present invention.

A process in each step of the disease label creation method illustrated in Fig. 9 is performed by the processor 12 and particularly corresponds to the process performed by the disease label creation device 10-1 according to the first embodiment illustrated in Fig. 2.

In Fig. 9, the information acquisition unit 28 of the processor 12 reads out the simple X-ray image 1 and the three-dimensional CT image 2 of the same patient and the three-dimensional first disease label extracted from the CT image 2 from the first database 16 (Step S10).

The registration processing unit 30 of the processor 12 performs the registration between the simple X-ray image 1 and the CT image 2 (Step S12). In this case, the registration processing unit 30 projects the CT image 2 according to the projection conditions (the posture parameters of the CT image 2 and geometric information) to create a DRR image and calculates the similarity between the simple X-ray image 1 (or a normalized simple X-ray image) and the DRR image. Then, the registration between the simple X-ray image 1 and the CT image 2 is performed by adjusting the projection conditions such that the calculated similarity is maximized.

The disease label converter 40 of the processor 12 converts the three-dimensional first disease label acquired together with the three-dimensional CT image 2 into a two-dimensional second disease label corresponding to the simple X-ray image 1 on the basis of the result of the registration in Step S12 (Step S14). That is, in Step S14, the second disease label is created by projecting the three-dimensional first disease label on the basis of the result (projection conditions) of the registration between the simple X-ray image 1 and the CT image 2 in the same manner as the three-dimensional CT image 2 to be converted into the two-dimensional disease label (second disease label) corresponding to the simple X-ray image 1.

The processor 12 stores a pair of the simple X-ray image 1 and the created second disease label as the first training data in the second database 18 (Step S16).

The processor 12 determines whether or not the creation of all of the disease labels based on the simple X-ray image 1, the three-dimensional CT image 2, and the like of the same patient stored in the first database 16 has been ended (Step S 18). In a case in which it is determined that the creation of all of the disease labels has not been ended (in the case of "NO"), the processor 12 proceeds to Step S10, reads out the simple X-ray image 1, the three-dimensional CT image 2, and the like of another patient, creates a disease label in the same manner as described above, and ends the process of automatically creating the disease label in a case in which it is determined that the creation of all of the disease labels has been ended (in the case of "YES").

### [Others]

In this embodiment, the case has been described in which the chest X-ray image is used as the simple X-ray image. However, the present invention is not limited thereto and can also be applied to other simple X-ray images such as abdominal X-ray images. In addition, the disease label created by the disease label creation device according to the present invention and the disease label detected by the disease detection model according to the present invention are not limited to the information indicating the region of the lung nodule and may be information indicating regions of other diseases.

Further, in this embodiment, the case has been described in which the Bayesian U-Net is used as the disease detection model. However, the disease detection model is not limited to the Bayesian U-Net, and any learning model may be used as long as it detects the disease region from the simple X-ray image.

In addition, in this embodiment, for example, the hardware structure of the processing unit that executes various processes, such as the CPU, is the following various processors. The various processors include, for example, a central processing unit (CPU) which is a general-purpose processor executing software (program) to function as various processing units, a programmable logic device (PLD), such as a field programmable gate array (FPGA), which is a processor whose circuit configuration can be changed after manufacture, and a dedicated electric circuit, such as an application specific integrated circuit (ASIC), which is a processor having a dedicated circuit configuration designed to perform a specific process.

One processing unit may be configured by one of these various processors or by two or more processors of the same type or different types (for example, a plurality of FPGAs or a combination of a CPU and an FPGA). Moreover, a plurality of processing units may be configured by one processor. A first example of the configuration in which a plurality of processing units are configured by one processor is an aspect in which one processor is configured by a combination of one or more CPUs and software and functions as a plurality of processing units. A representative example of this aspect is a client computer or a server computer. A second example of the configuration is an aspect in which a processor that implements the functions of the entire system including a plurality of processing units using one integrated circuit (IC) chip is used. A representative example of this aspect is a system-on-chip (SoC). As described above, various processing units are configured by using one or more of the various processors as a hardware structure.

In addition, more specifically, the hardware structure of these various processors is an electric circuit (circuitry) obtained by combining circuit elements such as semiconductor elements.

Further, the present invention includes a disease label creation program that is installed in a computer and causes the computer to function as the disease label creation device according to the present invention and a non-volatile storage medium in which the disease label creation program is recorded.

Furthermore, it is needless to say that the present invention is not limited to the above-described embodiments and various modifications can be made without departing from the gist of the present invention.

### Explanation of References

1, 5: simple X-ray image
2: CT image
3-1, 3-2: disease label
3A, 3B: rectangular region
4-1, 4-2, 6-1, 6-2: detection result
6A, 6B: lung nodule
10, 10-1, 10-2: disease label creation device
11-1, 11-2: learning device
12: processor
14: memory
16: first database
18: second database
20: display
22: input/output interface
24: operation unit
28: information acquisition unit
30: registration processing unit
31: normalization unit
32: comparison unit
33: DRR image creation unit
34: geometric information
35: optimization unit
38: X-ray source position
39: detector position
40: disease label converter
42: reliability calculator
50-1, 50-2, 52-1, 52-2: disease detection model
60-1, 60-2: learning processing unit
62-1, 62-2: error calculation unit
64: parameter control unit
S10 to S18: step

## Claims

1. A disease label creation device comprising:
a first processor,
wherein the first processor is configured to execute:
an information acquisition process of acquiring a simple X-ray image, a three-dimensional CT image paired with the simple X-ray image, and a three-dimensional first disease label extracted from the CT image;
a registration process of performing registration between the simple X-ray image and the CT image; and
a conversion process of converting the first disease label into a two-dimensional second disease label corresponding to the simple X-ray image on the basis of a result of the registration.

2. The disease label creation device according to claim 1,
wherein the registration process includes:
a process of projecting the CT image to create a pseudo X-ray image; and
a process of performing registration between the simple X-ray image and the pseudo X-ray image.

3. The disease label creation device according to claim 1,
wherein the registration process includes:
a process of extracting a two-dimensional anatomical landmark from the simple X-ray image;
a process of extracting a three-dimensional anatomical landmark corresponding to the anatomical landmark from the CT image;
a process of projecting the three-dimensional anatomical landmark; and
a process of performing registration between the two-dimensional anatomical landmark and an anatomical landmark after the projection process.

4. The disease label creation device according to claim 1,
wherein the registration process includes:
a process of extracting a two-dimensional anatomical region of interest from the simple X-ray image;
a process of extracting a three-dimensional anatomical region of interest corresponding to the anatomical region of interest from the CT image;
a process of projecting the three-dimensional anatomical region of interest; and
a process of performing registration between a contour of the two-dimensional anatomical region of interest and a contour of an anatomical region of interest after the projection process.

5. The disease label creation device according to claim 1,
wherein the registration process includes:
a process of three-dimensionally restoring the simple X-ray image; and
a process of performing registration between the CT image and the three-dimensionally restored simple X-ray image.

6. The disease label creation device according to any one of claims 1 to 5,
wherein the first processor is configured to:
execute a first reliability calculation process of calculating a first reliability for the second disease label.

7. The disease label creation device according to claim 6,
wherein, in the first reliability calculation process, a visibility of a second disease region corresponding to the second disease label with respect to a normal region of the simple X-ray image is calculated using at least one of statistics of pixel values of a normal region and a first disease region of the CT image corresponding to the first disease label or a shape feature of the first disease region of the CT image, and the first reliability is calculated from the calculated visibility.

8. The disease label creation device according to claim 6,
wherein, in the information acquisition process, information of an anatomical region in the CT image is acquired, and
in the first reliability calculation process, a visibility of a second disease region corresponding to the second disease label with respect to a normal region of the simple X-ray image is calculated on the basis of superimposition of the anatomical region and a first disease region of the CT image corresponding to the first disease label in a projection direction, and the first reliability is calculated from the calculated visibility.

9. The disease label creation device according to claim 6,
wherein the first disease label is a label automatically detected from the CT image,
in the information acquisition process, an interpretation report corresponding to the simple X-ray image or the CT image is acquired, and
in the first reliability calculation process, the first reliability is calculated on the basis of a rate of match between the first disease label and content described in the interpretation report.

10. The disease label creation device according to claim 6,
wherein the first processor is configured to:
calculate a degree of success of the result of the registration, and
in the first reliability calculation process, the first reliability is calculated on the basis of the degree of success.

11. The disease label creation device according to claim 6,
wherein the first disease label is a label automatically detected from the CT image, and
in the first reliability calculation process, a low first reliability is given to the second disease label of a region having different imaging ranges in the simple X-ray image and the CT image forming the pair.

12. The disease label creation device according to any one of claims 1 to 11,
wherein, in the registration process, the registration is performed by adjusting a solution space in the registration between the simple X-ray image and the CT image forming the pair associated with a patient, depending on the patient.

13. The disease label creation device according to claim 12, further comprising:
a database of a statistical deformation model for each patient feature information item,
wherein the registration process includes:
a process of selecting a corresponding statistical deformation model from the database on the basis of patient feature information of the patient corresponding to the simple X-ray image and the CT image forming the pair; and
a process of performing non-rigid registration between the simple X-ray image and the CT image using the selected statistical deformation model.

14. The disease label creation device according to any one of claims 1 to 13,
wherein, in the information acquisition process, an image-level third disease label of the CT image is acquired, and
the first processor is configured to:
give the second disease label and the third disease label to the simple X-ray image.

15. The disease label creation device according to any one of claims 1 to 14,
wherein, in the information acquisition process, an image-level third disease label of the CT image is acquired, and
the first processor is configured to:
determine whether the result of the registration is a success or a failure;
select the second disease label in a case in which it is determined that the result is a success and select the third disease label in a case in which it is determined that the result is a failure; and
give the selected second disease label or the selected third disease label to the simple X-ray image.

16. A disease label creation method executed by a processor, the disease label creation method comprising:
a step of acquiring a simple X-ray image, a three-dimensional CT image paired with the simple X-ray image, and a three-dimensional first disease label extracted from the CT image;
a step of performing registration between the simple X-ray image and the CT image; and
a step of converting the first disease label into a two-dimensional second disease label corresponding to the simple X-ray image on the basis of a result of the registration.

17. A disease label creation program causing a computer to implement:
a function of acquiring a simple X-ray image, a three-dimensional CT image paired with the simple X-ray image, and a three-dimensional first disease label extracted from the CT image;
a function of performing registration between the simple X-ray image and the CT image; and
a function of converting the first disease label into a two-dimensional second disease label corresponding to the simple X-ray image on the basis of a result of the registration.

18. A non-transitory computer-readable recording medium on which the program according to claim 17 is recorded.

19. A learning device comprising:
a second processor,
wherein the second processor is configured to:
execute a learning process of training a disease detection model, using first training data consisting of the simple X-ray image and the second disease label created by the disease label creation device according to claim 1 or 2 and converging a first error between an output of the disease detection model and the second disease label.

20. A learning device comprising:
a second processor,
wherein the second processor is configured to:
in a case in which a learning process of training a disease detection model, using second training data consisting of the simple X-ray image, the second disease label created by the disease label creation device according to any one of claims 6 to 11, and the first reliability and converging a first error between an output of the disease detection model and the second disease label is performed, execute the learning process of adjusting the first error according to the first reliability to train the disease detection model.

21. The learning device according to claim 19 or 20,
wherein, in the information acquisition process, an image-level third disease label of the CT image is acquired,
the first processor is configured to:
give the second disease label and the third disease label to the simple X-ray image, and
the second processor is configured to:
execute a learning process of converging a second error between the output of the disease detection model and the third disease label, using the simple X-ray image to which the third disease label has been given as third training data.

22. The learning device according to claim 20,
wherein the second processor is configured to:
execute a learning process of directing the disease detection model to output a disease detection result indicating a disease region included in the simple X-ray image and a second reliability of the disease detection result.

23. The learning device according to claim 22,
wherein the second processor is configured to:
adjust the first error of the disease region, of which the second reliability output from the disease detection model is low and which is false positive, to a large value and adjust the first error of the disease region, of which the second reliability is low and which is false negative, to a small value.

24. The learning device according to claim 22 or 23,
wherein the second processor is configured to:
in a case in which a learning process of integrating the first reliability calculated by the first reliability calculation process and the second reliability output from the disease detection model to generate a third reliability and converging a first error between an output of the disease detection model and the second disease label, execute the learning process of adjusting the first error according to the third reliability to train the disease detection model.

25. A disease detection model trained by the learning device according to any one of claims 19 to 24,
wherein the disease detection model receives any simple X-ray image as an input image, detects a disease label from the input simple X-ray image, and outputs the disease label.
